(19) **Europäisches Patentamt**

**European Patent Office**

**Office européen des brevets**

(11) **EP 0 639 550 B1**

(12) **EUROPEAN PATENT SPECIFICATION**

(45) Date of publication and mention
of the grant of the patent:
**06.09.2000 Bulletin 2000/36**

(51) Int. Cl.[7]: **C07C 11/107**, C07C 5/25,
C07C 2/30

(21) Application number: **94908492.5**

(22) Date of filing: **04.03.1994**

(86) International application number:
**PCT/JP94/00346**

(87) International publication number:
**WO 94/20439 (15.09.1994 Gazette 1994/21)**

(54) **PROCESS FOR PRODUCING 2,3-DIMETHYL-2-BUTENE**

VERFAHREN ZUR HERSTELLUNG VON 2, 3-DIMETHYL-2-BUTEN

PROCEDE DE PRODUCTION DE 2,3-DIMETHYL-2-BUTENE

(84) Designated Contracting States:
**AT BE CH DE DK FR GB IT LI NL SE**

(30) Priority: **04.03.1993 JP 4384393**
**28.07.1993 JP 18618293**

(43) Date of publication of application:
**22.02.1995 Bulletin 1995/08**

(73) Proprietor:
**SUMITOMO CHEMICAL COMPANY LIMITED**
**Osaka-shi, Osaka 541-0041 (JP)**

(72) Inventors:
• **NOMURA, Kotohiro**
**Takatsuki-shi, Osaka 569 (JP)**
• **YAMAMOTO, Michio**
**Otsu-shi, Shiga 520-02 (JP)**

• **KOMATSU, Masashi**
**Ibaraki-shi, Osaka 567 (JP)**

(74) Representative:
**VOSSIUS & PARTNER**
**Siebertstrasse 4**
**81675 München (DE)**

(56) References cited:
**JP-A- 55 147 231**      **JP-A- 59 219 240**
**JP-A- 63 280 031**

Remarks:
The file contains technical information submitted
after the application was filed and not included in
this specification

**Description**

The present invention relates to a process for preparing 2,3-dimethyl-2-butene (hereinafter referred to as "TMEN"). In particular, the present invention relates to a process for preparing TMEN by isomerizing 2,3-dimethyl-1-butene (hereinafter referred to as "DMB-1").

TMEN is an important compound as a basic intermediate for agrochemicals, medicines, perfumes, cosmetic materials, and so on. For the preparation thereof, there is known a process comprising contacting DMB-1 which is synthesized by dimerization of propene with a following solid or liquid isomerization catalyst to convert DMB-1 to TMEN:

(1) A solid base catalyst such as Na-Al$_2$O$_3$ (cf. Japanese Patent KOKAI Publication Nos. 196526/1988, 196527/1988, 8707/1973, and the like),
(2) Sulfonated divinylbenzene-styrene copolymer (cf. German Patent No. 2,449,298, Japanese Patent KOKAI Publication No. 280031/1988, and the like),
(3) Molecular sieves 5A or 5-13A (cf. US Patent No. 3,636,124, German Patent No. 2,055,478, and the like),
(4) AlEt$_3$/1,1,1,3,3,3-hexafluoroisopropyl alcohol catalyst (cf. Japanese Patent KOKAI Publication No. 209028/1987, and the like).

However, in a case where the above solid catalysts (1), (2) and (3) are used as the isomerization catalysts, when DMB-1 is isomerized without separating a dimerization catalyst from the dimerization reaction product, an isomerization activity and a life of the catalyst are deteriorated because the solid catalyst may absorb the dimerization catalyst. Therefore, the dimerization catalyst should be removed from the dimerization reaction product.

The process which uses the liquid isomerization catalyst (4) has a drawback that an expensive alcohol is used as a catalyst component.

An object of the present invention is to provide a process for preparing TMEN from DMB-1 using the reaction product from the dimerization of propene in the isomerization process without removing the dimerization catalyst from the dimerization reaction product.

According to a first aspect of the present invention, there is provided a process for preparing TMEN comprising isomerizing DMB-1 at a temperature of -70°C to +150°C in the presence of at least one acid selected from sulfuric acid having a concentration of at least 70% and sulfonic acids wherein the amount of the sulfuric acid or sulfonic acid is from 0.01 to 5.0 wt% based on DMB-1.

According to a second aspect of the present invention, there is provided a process for preparing 2,3-dimethyl-2-butene comprising forming 2,3-dimethyl-1-butene from propene in the presence of a dimerization catalyst and isomerizing formed 2,3-dimethyl-1-butene in the presence of at least one acid selected from sulfuric acid having a concentration of at least 70% and sulfonic acids.

The present invention will be explained in detail.

When DMB-1 is isomerized to prepare TMEN, sulfuric acid, a sulfonic acid or their mixture is used as the isomerization catalyst.

Specific examples of the sulfonic acid are aliphatic sulfonic acids such as methanesulfonic acid, ethanesulfonic acid, etc.; aromatic sulfonic acids such as benzenesulfonic acid, p-toluenesulfonic acid, etc.; halogenated sulfonic acids such as chlorosulfonic acid, etc., and mixtures thereof. It is possible to use a mixture of sulfuric acid and at least one sulfonic acid. Among sulfuric acid and the sulfonic acids, sulfuric acid and methanesulfonic acid are preferred. In particular, cheap sulfuric acid is technically advantageous.

The concentration of sulfuric acid to be used is at least 70 %, preferably 90 to 98 %. The amount of sulfuric acid depends on its concentration, and is from 0.01 to 5.0 wt. %, preferably from 0.05 to 3.0 wt. %, more preferably from 0.05 to 1.5 wt. % based on DMB-1.

The amount of the sulfonic acid is also from 0.01 to 5.0 wt. %, preferably from 0.05 to 3.0 wt. %, more preferably from 0.05 to 1.5 wt. % based on DMB-1.

The isomerization reaction is usually carried out in an inactive solvent. As the inactive solvent, there are used aromatic hydrocarbons such as benzene, toluene, xylene, etc.; aliphatic hydrocarbons such as hexane, heptane, cyclohexane, etc.; halogenated hydrocarbons such as dichloromethane, dichloroethane, chloroform, chlorobenzene, dichlorobenzene, etc., and the like.

An amount of the inactive solvent is usually from 0.001 to 50 parts by weight, preferably from 0.001 to 10 parts by weight, more preferably from 0.001 to 1 part by weight per one part by weight of DMB-1.

The temperature in the isomerization reaction is from -30 to +100°C, preferably from 0 to 60°C.

As explained above, DMB-1 is isomerized to TMEN. TMEN can be recovered by, for example, adding an amine or an aqueous solution of an alkali to the reaction mixture after the isomerization, mixing them, separating an oil layer and distilling the oil layer.

DMB-1 which is a raw material in the process of the present invention is generally prepared by dimerizing propene using a catalyst. The dimerization catalyst is not limited insofar as DMB-1 can be formed. Specific examples of such catalyst are the following catalysts based on a nickel compound:

(1) a catalyst based on π-Allyl nickel complex/organic aluminum halide/trivalent phosphorus compound (Japanese Patent Publication No. 34007/1971, and so on),
(2) a catalyst based on nickel salt/organic aluminum halide/trivalent phosphorus compound (cf. Japanese Patent Publication No. 22807/1972, and so on),
(3) a catalyst based on nickel compound/trialkylaluminum/trivalent phosphorus compound/halogenated phenol/water (cf. Japanese Patent KOKAI Publication No. 167932/1982, and so on),
(4) a catalyst based on nickel compound/trialkylaluminum/trivalent phosphorus compound/fluorinated isopropyl alcohol (cf. Japanese Patent KOKAI Publication Nos. 156225/1987 and 221335/1989, and so on),
(5) a catalyst based on nickel compound/trialkylaluminum/trivalent phosphorus compound/halogenated phenol/sulfonic acid or dialkyl sulfate (cf. Japanese Patent Application No. 346027/1992, and so on),
(6) a catalyst based on nickel compound/trialkylaluminum/trivalent phosphorus compound/fluorinated isopropyl alcohol/sulfonic acid or dialkyl sulfate (cf. Japanese Patent Application No. 270087/1992 and so on),
(7) a catalyst based on nickel compound/trialkylaluminum/trivalent phosphorus compound/halogenated phenol/water/sulfonic acid (cf. Japanese Patent Application No. 147202/1992, and so on).

In general, the dimerization reaction is carried out in an inactive solvent. As the inactive solvent, the same solvents as used in the above isomerization process can be exemplified.

When the dimerization catalyst described above is used, a concentration of the catalyst is usually from about $10^{-5}$ to $10^{-1}$ mol/l in terms of a concentration of nickel.

The temperature in the dimerization reaction is from -70 to +150°C, preferably from -50 to +100°C, more preferably from -20 to +50°C. A pressure in the dimerization reaction is usually from 0 to 30 kg/cm$^2$G.

In the above manner, the dimerization reaction mixture containing DMB-1 is obtained from propene. This mixture can be supplied to the above isomerization step without removing the dimerization catalyst. Needless to say, it is possible to supply this mixture to the isomerization step after removing the catalyst by, for example, distillation and so on.

When the mixture is supplied without removing the dimerization catalyst, a yield of 2,3-dimethylbutenes (DMB-1 and TMEN) can be increased by carrying out the isomerization reaction after deactivating the dimerization catalyst by the addition of a deactivating agent such as alcohols, phenols, ammonia, amines, for example, alkylamines such as triethylamine to the dimerization reaction mixture.

The alcohol to be added is not limited. Specific examples are methanol, ethanol, n-propyl alcohol, isopropyl alcohol, n-butyl alcohol, isobutyl alcohol, tert.-butyl alcohol, and the like. Among them, isopropyl alcohol is preferably used.

As the phenols, phenol and alkyl-substituted phenols are exemplified. Examples of the alkyl-substituted phenols are o-cresol, m-cresol, p-cresol, 2,6-di-tert.-butyl-4-methylphenol (hereinafter referred to as "BHT"), and the like. Among them, BHT and p-cresol are preferably used.

An amount of the alcohol and phenol is usually from 0.01 to 10 wt. %, preferably from 0.01 to 5 wt. % based on the raw material propene.

To deactivate the dimerization catalyst, in addition to the above deactivating agent, it is possible to use an aqueous deactivating agent such as aqueous ammonia, an aqueous solution of amines, an aqueous solution of inorganic strong bases (e.g. aqueous sodium hydroxide, aqueous potassium hydroxide, etc.), an aqueous solution of mineral acids (e.g. dilute hydrochloric acid, etc.), and water. When the dimerization catalyst is deactivated by the aqueous deactivation agent, the isomerization catalyst is usually carried out after removing an aqueous layer from an oil layer containing DMB-1 and so on.

According to the present invention, TMEN can be effectively prepared from DMB-1 using a cheap catalyst such as sulfuric acid or sulfonic acids. Also, the process of the present invention is technically advantageous since the dimerization reaction mixture from propene can be used in the isomerization process even if the dimerization catalyst is not removed.

EXAMPLES

The present invention will be illustrated more in detail by the following examples, which do not limit the scope of the present invention.

Reference Example 1 (preparation of catalyst solution)

In a 50 ml Schlenk tube which had been replaced by nitrogen and cooled to 5°C, toluene (1.35 ml) containing nickel naphthenate (0.1 mmol), tricyclohexylphosphine (0.1 mmol, 20 % toluene solution) and isoprene (8 mmol) were charged at the same temperature. After adding toluene (1 ml) containing triethylaluminum (1.0 mmol), the mixture was warmed to 18°C while stirring.

After cooling to 5°C, trifluoromethanesulfonic acid (0.1 mmol) was added and then toluene (1.5 ml) containing 1,1,1,3,3,3-hexafluoroisopropyl alcohol (1.5 mmol) was added with maintaining an internal temperature not exceeding 20°C to obtain a catalyst solution.

Example 1

In a 1500 ml stainless steel autoclave which had been replaced by nitrogen, the catalyst solution obtained in Reference Example 1 and toluene (40 ml) were charged at 15°C, and propene was supplied under pressure of 3 kg/cm$^2$G and reacted at 10°C for 2 hours. After cooling the mixture to 5°C, unreacted propene was discharged outside the system.

The reaction mixture was analyzed by gas chromatography to find that a produced amount of DMB-1 was 3821 mmol, and TMEN was not detected. The selectivity of the dimers was 74.5 %, and a ratio of DMB-1 in the dimers was 78.4 %. T.O.N. of DMB-1 was 38210.

To the above reaction mixture, 90 % sulfuric acid was added in an amount of 0.4 wt. % based on the raw material propene, and the reaction was effected at 30°C for 3 hours. The reaction mixture was analyzed by gas chromatography, and the results are shown in Table 1.

Dimers: DMB-1, TMEN, 2M1P, 2M2P, 4M1,2P and Hex.
(DMB-1: 2,3-dimethyl-1-butene, TMEN: 2,3-dimethyl-2-butene, 2M1P: 2-methyl-1-pentene, 2M2P: 2-methyl-2-pentene, 4M1,2P: 4-methyl-1-pentene and 4-methyl-2-pentene, Hex: hexene)

Selectivity of dimers (%) = [Amount of produced dimers (g)/Amount of reacted propene (g)] x 100

T.O.N. (turnover number) = Produced amount (mmol)/Nickel amount in catalyst (mmol)

Examples 2-6

Under the same conditions as in Example 1 except that a concentration and an amount of sulfuric acid were changed as shown in Table 1, the reaction was effected.

After the reaction, the resulting reaction mixture was analyzed by gas chromatography. The results are shown in Table 1.

## Table 1

| Exam- ple No. | Sulfuric acid Concent- ration (%) | Amount[1) (wt. %) | TMEN ratio[2) (%) | Recovery rate[3) (%) |
|---|---|---|---|---|
| Ex. 1 | 90 | 0.4 | 91.0 | 96.2 |
| Ex. 2 | 90 | 0.35 | 89.9 | 96.1 |
| Ex. 3 | 93 | 0.1 | 89.6 | 97.0 |
| Ex. 4 | 93 | 0.2 | 90.6 | 94.9 |
| Ex. 5 | 93 | 0.3 | 91.6 | 94.2 |
| Ex. 6 | 95 | 0.2 | 88.2 | 99.3 |

1) Amount: Weight % based on the raw material propene.

2) TMEN ratio (%) = Ratio of TMEN in 2,3-dimethylbutenes after the isomerization reaction.

= [Amount of TMEN (mmol) after the isomerization reaction/Amounts of 2,3-dimethylbutenes (mmol) after the isomerization reaction] x 100.

3) Recovery rate (%) = Recovery rate (%) of 2,3-dimethylbutenes after the isomerization reaction

= [(Amounts of 2,3-dimethylbutenes (mmol) after the isomerization reaction)/(Amounts of 2,3-dimethylbutenes (mmol) before the isomerization reaction)] x 100.

Examples 7 and 8

Under the same conditions as in the Example 1 except that the amount of sulfuric acid, and a temperature and a time of the isomerization reaction were changed as shown in Table 2, the reaction was carried out.

After the reaction, the resulting reaction mixture was analyzed by gas chromatography. The results are shown in Table 2.

Table 2

| Example No. | Sulfuric acid | | Isomerization reaction | | TMEN ratio (%) | Recovery rate (%) |
|---|---|---|---|---|---|---|
| | Concentration (%) | Amount (wt. %) | Temp (°C) | Time (hr) | | |
| Ex. 7 | 90 | 0.5 | 40 | 2 | 91.5 | 93.2 |
| Ex. 8 | 90 | 0.3 | 40 | 2 | 88.1 | 94.3 |

Comparative Example 1

Under the same conditions as in Example 8 except that AMBERLIST 15 (a strongly acidic cation exchange resin manufactured by Rohm & Haas Co., USA) in an amount of 1.0 wt. % based on the raw material propene was used in place of sulfuric acid, the reaction was carried out.

After the reaction, the resulting reaction mixture was analyzed by gas chromatography. The results are shown in Table 3.

Table 3

| Example No. | Sulfuric acid | | Amount of AMBERLIST 15 (wt. %) | TMEN ratio (%) |
|---|---|---|---|---|
| | Concentratio (%) | Amount (wt. %) | | |
| Ex. 8 | 90 | 0.3 | --- | 88.1 |
| C. Ex. 1 | -- | --- | 1.0 | 51.7 |

Example 9

Under the same conditions as in Example 1 except that methanesulfonic acid was used in an amount of 0.75 wt. % based on the raw material propene in place of sulfuric acid and the reaction was carried out at 40°C for 3 hours, the reaction was effected.

After the reaction, the resulting reaction mixture was analyzed by gas chromatography. The TMEN ratio in the 2,3-dimethylbutenes after the isomerization reaction was 88.2 %, and the recovery rate of the 2,3-dimethylbutenes was 99.3 %.

Example 10

Under the same conditions as in Example 1, propene was dimerized. To the obtained reaction mixture, 2,6-di-tert.-butyl-4-methylphenol (BHT) was added in an amount of 0.2 wt. % based on the raw material propene at 25°C followed by stirring for 5 minutes. To the mixture, 90 % sulfuric acid was added in an amount of 0.35 wt. % based on the raw material propene and the isomerization reaction was carried out at 30°C for 3 hours. After the reaction, the reaction mixture was analyzed by gas chromatography. The results are shown in Table 4.

Examples 11 to 15

Under the same conditions as in Example 10 except that a phenol compound of Table 4 was used in an amount shown in Table 4 instead of using 2,6-di-tert.-butyl-4-methylphenol (BHT) in an amount of 0.2 wt. % based on the raw material propene, and a concentration and an amount of sulfuric acid were changed as shown in Table 4, the reaction was effected.

After the reaction, the reaction mixture was analyzed by gas chromatography. The results are shown in Table 4.

Table 4

| Exam. No. | Phenol Kind | Amount[1] | Sulfuric acid | | TMEN ratio (%) | Recovery rate (%) |
|---|---|---|---|---|---|---|
| | | | Conc. | Amount[1] | | |
| Ex. 10 | BHT | 0.2 | 90 % | 0.35 | 92.7 | 97.2 |
| Ex. 11 | BHT | 0.2 | 90 % | 0.50 | 93.2 | >99.9 |
| Ex. 12 | BHT | 0.1 | 90 % | 0.50 | 91.6 | 99.6 |
| Ex. 13 | p-Cresol | 0.2 | 90 % | 0.50 | 90.9 | >99.9 |
| Ex. 14 | Phenol | 0.2 | 90 % | 0.50 | 90.3 | 96.8 |
| Ex. 15 | BHT | 0.2 | 93 % | 0.30 | 91.0 | 98.3 |

1) Amount: Weight % based on the raw material propene.

Example 16

Under the same conditions as in Example 1, propene was dimerized. To the obtained reaction mixture, isopropyl alcohol (0.5 ml) was added at 25°C followed by stirring for 5 minutes. To the mixture, 90 % sulfuric acid was added in an amount of 0.30 wt. % based on the raw material propene and the isomerization reaction was carried out at 40°C for 2 hours. After the reaction, the reaction mixture was analyzed by gas chromatography. The TMEN ratio in the 2,3-dimethylbutenes was 90.3 %, and the recovery rate of the 2,3-dimethylbutenes was 97.4 % or higher.

Example 17

In a 1500 ml stainless steel autoclave which had been replaced by nitrogen, the catalyst solution obtained in Reference Example 1 and toluene (40 ml) were charged at 15°C, and propene was supplied under pressure of 3 kg/cm$^2$G and reacted at 10°C for 2 hours. After cooling the mixture to 5°C, unreacted propene was discharged outside the system.

To the reaction mixture, a 2 % aqueous solution of sodium hydroxide was added in an amount of 50 wt. % based on the reaction liquid, followed by stirring at 40°C for 30 minutes. After cooling it to 30°C, the mixture was kept standing and phase separated to obtain an oily layer. The oily layer was analyzed by gas chromatography to find that an amount of the dimers in the oily layer was 98 % or higher of the whole dimers formed by the reaction.

One hundred grams of the obtained oily layer was charged in a 500 ml autoclave, and 95 % sulfuric acid (0.7 g) was dropwise added thereto over a period of 30 minutes with keeping the temperature of the reaction mixture not exceeding 30°C. The obtained reaction mixture was analyzed by gas chromatography to find that the TMEN ratio in the 2,3-dimethylbutenes after the isomerization reaction was 92.5 %, and the recovery of the 2,3-dimethylbutenes was 99 %.

Examples 18 to 24

Under the same conditions as in Example 17 except that a concentration and an amount of sulfuric acid and a reaction temperature were changed as shown in Table 5, the reaction was effected. The results are shown in Table 5.

Table 5

| Exam. No. | Sulfuric acid | | Reaction temp. (°C) | TMEN ratio (%) | Recovery rate (%) |
|---|---|---|---|---|---|
| | Conc. (%) | Amount (g) | | | |
| Ex. 17 | 95 | 0.7 | 30 | 92.5 | 99 |
| Ex. 18 | 93 | 0.7 | 30 | 90.9 | 99 |
| Ex. 19 | 93 | 1.0 | 30 | 91.5 | 99 |
| Ex. 20 | 93 | 1.0 | 40 | 92.0 | 99 |
| Ex. 21 | 95 | 0.5 | 30 | 93.1 | 99 |

Table 5 (continued)

| Exam. No. | Sulfuric acid | | Reaction temp. (°C) | TMEN ratio (%) | Recovery rate (%) |
| --- | --- | --- | --- | --- | --- |
| | Conc. (%) | Amount (g) | | | |
| Ex. 22 | 95 | 0.8 | 30 | 92.9 | 99 |
| Ex. 23 | 95 | 1.0 | 30 | 92.3 | 99 |
| Ex. 24 | 97 | 0.7 | 30 | 91.7 | 99 |

Comparative Example 2

Under the same conditions as in Example 20 except that AMBERLIST 15 (1.0 g) was used in place of sulfuric acid, the reaction was carried out.

After the reaction, the reaction mixture was analyzed by gas chromatography. The results are shown in Table 6.

Table 6

| Exam. No. | 93 % sulfuric acid (g) | AMBERLIST 15 (g) | TMEN ratio (%) |
| --- | --- | --- | --- |
| Ex. 20 | 1.0 | --- | 92.0 |
| C. Ex. 2 | --- | 1.0 | 46.8 |

**Claims**

1.   A process for preparing 2,3-dimethyl-2-butene comprising isomerizing 2,3-dimethyl-1-butene at a temperature of -30°C to +100°C in the presence of at least one acid selected from sulfuric acid having a concentration of at least 70% and sulfonic acids wherein the amount of the sulfuric acid or sulfonic acid is from 0.01 to 5.0 wt% based on 2,3-dimethyl-1-butene.

2.   A process according to claim 1 for preparing 2,3-dimethyl-2-butene comprising forming 2,3-dimethyl-1-butene from propene in the presence of a dimerization catalyst and isomerizing formed 2,3-dimethyl-1-butene in the presence of at least one acid selected from sulfuric acid having a concentration of at least 70% and sulfonic acids.

3.   The process according to claim 1 or 2, wherein said sulfonic acid is at least one sulfonic acid selected from aliphatic sulfonic acids, aromatic sulfonic acids and halogenated sulfonic acids.

4.   The process according to claim 3, wherein the aromatic sulfonic acid is benzenesulfonic acid or p-toluenesulfonic acid.

5.   The process according to claim 1 or 2, wherein said acid is used in an amount of 0.001 to 5 wt. % based on 2,3-dimethyl-1-butene.

6.   The process according to claim 1 or 2, wherein at least one solvent selected from aromatic hydrocarbons, aliphatic hydrocarbons and halogenated hydrocarbons is used.

7.   The process according to claim 6, wherein an amount of said solvent is from 0.01 to 50 parts by weight per one part of 2,3-dimethyl-1-butene.

8.   The process according to claim 1 or 2, wherein an isomerization reaction temperature is from -30 to 100°C.

9.   The process according to claim 1 or 2, wherein the process is carried out in the presence of sulfuric acid having a concentration of at least 70%.

10.  The process according to claim 2, wherein said dimerization catalyst is deactivated after dimerization of propene to form 2,3-dimethyl-1-butene and then 2,3-dimethyl-1-butene is isomerized.

11. The process according to claim 2 or 10, wherein said dimerization catalyst is a homogeneous catalyst.

12. The process according to claim 11, wherein said homogeneous catalyst is at least one catalyst, which comprises a nickel compound, selected from the group consisting of (1) a catalyst based on $\pi$-allyl nickel complex/organic aluminum halide/trivalent phosphorus compound, (2) a catalyst based on nickel salt/organic aluminum halide/trivalent phosphorus compound, (3) a catalyst based on nickel compound/trialkylaluminum/trivalent phosphorus compound/halogenated phenol/water, (4) a catalyst based on nickel compound/trialkylaluminum/trivalent phosphorus compound/fluorinated isopropyl alcohol, (5) a catalyst based on nickel compound/trialkylaluminum/trivalent phosphorus compound/halogenated phenol/sulfonic acid or dialkyl sulfate, (6) a catalyst based on nickel compound/trialkylaluminum/trivalent phosphorus compound/fluorinated isopropyl alcohol/sulfonic acid or dialkyl sulfate and (7) a catalyst based on nickel compound/trialkylaluminum/trivalent phosphorus compound/halogenated phenol/water/sulfonic acid.

13. The process according to claim 10, wherein a deactivator of said catalyst is at least one compound selected from alcohols, phenols, ammonia and amines.

**Patentansprüche**

1. Verfahren zur Herstellung von 2,3-Dimethyl-2-buten, umfassend Isomerisieren von 2,3-Dimethyl-1-buten bei einer Temperatur von -30 °C bis +100 °C in Gegenwart wenigstens einer Säure, ausgewählt aus Schwefelsäure mit einer Konzentration von wenigstens 70% und Sulfonsäuren, wobei die Menge an Schwefelsäure oder Sulfonsäure 0,01 bis 5,0 Gew.%, bezogen auf 2,3-Dimethyl-1-buten, beträgt.

2. Verfahren nach Anspruch 1 zur Herstellung von 2,3-Dimethyl-2-buten, umfassend Erzeugen von 2,3-Dimethyl-1-buten aus Propen in Gegenwart eines Dimerisierungskatalysators und Isomerisieren des erzeugten 2,3-Dimethyl-1-butens in Gegenwart wenigstens einer Säure, ausgewählt aus Schwefelsäure mit einer Konzentration von wenigstens 70% und Sulfonsäuren.

3. Verfahren nach Anspruch 1 oder 2, wobei die Sulfonsäure wenigstens eine aus aliphatischen, aromatischen und halogenierten Sulfonsäuren ausgewählte Sulfonsäure ist.

4. Verfahren nach Anspruch 3, wobei die aromatische Sulfonsäure Benzolsulfonsäure oder p-Toluolsulfonsäure ist.

5. Verfahren nach Anspruch 1 oder 2, wobei die Säure in einer Menge von 0,001 bis 5 Gew.%, bezogen auf 2,3-Dimethyl-1-buten, verwendet wird.

6. Verfahren nach Anspruch 1 oder 2, wobei wenigstens ein aus aromatischen, aliphatischen und halogenierten Kohlenwasserstoffen ausgewähltes Lösungsmittel verwendet wird.

7. Verfahren nach Anspruch 6, wobei die Menge des Lösungsmittels 0,01 bis 50 Gewichtsteile pro einem Teil 2,3-Dimethyl-1-buten beträgt.

8. Verfahren nach Anspruch 1 oder 2, wobei die Reaktionstemperatur bei der Isomerisierung -30 bis 100 °C beträgt.

9. Verfahren nach Anspruch 1 oder 2, wobei das Verfahren in Gegenwart von Schwefelsäure mit einer Konzentration von wenigstens 70% durchgeführt wird.

10. Verfahren nach Anspruch 2, wobei der Dimerisierungskatalysator nach der Dimerisierung von Propen zu 2,3-Dimethyl-1-buten deaktiviert wird und dann 2,3-Dimethyl-1-buten isomerisiert wird.

11. Verfahren nach Anspruch 2 oder 10, wobei der Dimerisierungskatalysator ein homogener Katalysator ist.

12. Verfahren nach Anspruch 11, wobei der homogene Katalysator wenigstens ein eine Nickelverbindung umfassender Katalysator ist, ausgewählt aus (1) einem Katalysator, basierend auf $\pi$-Allyl-Nickel-Komplex/organisches Aluminiumhalogenid/dreiwertige Phosphorverbindung, (2) einem Katalysator, basierend auf Nickelsalz/organisches Aluminiumhalogenid/dreiwertige Phosphorverbindung, (3) einem Katalysator, basierend auf Nickelverbindung/Trialkylaluminium/dreiwertige Phosphorverbindung/halogeniertes Phenol/Wasser, (4) einem Katalysator, basierend auf Nickelverbindung/Trialkylaluminium/dreiwertige Phosphorverbindung/fluorierter Isopropylalkohol, (5) einem Katalysator, basierend auf Nickelverbindung/Trialkylaluminium/dreiwertige Phosphorverbindung/halogeniertes Phenol/Sulfonsäure oder Dialkylsulfat, (6) einem Katalysator, basierend auf Nickelverbindung/Trialkylaluminium/dreiwertige Phosphorverbindung/fluorierter Isopropylalkohol/Sulfonsäure oder Dialkylsulfat, und (7) einem Katalysator, basierend auf Nickelverbindung/Trialkylaluminium/dreiwertige Phosphorverbindung/halogeniertes Phenol/Wasser/Sulfonsäure.

13. Verfahren nach Anspruch 10, wobei der Deaktivator für den Katalysator wenigstens eine aus Alkoholen, Phenolen, Ammoniak und Aminen ausgewählte Verbindung ist.

**Revendications**

1. Procédé pour la préparation de 2,3-diméthyl-2-butène comprenant l'isomérisation de 2,3-diméthyl-1-butène à une température de -30°C à + 100°C en présence d'au moins un acide choisi parmi l'acide sulfurique ayant une concentration d'au moins 70% et des acides sulfoniques, dans lequel la quantité de l'acide sulfurique ou de l'acide sulfonique est comprise entre 0,01 à 5,0 % en poids, rapportés au 2,3-diméthyl-1-butène.

2. Procédé selon la revendication 1 pour la préparation de 2,3-diméthyl-2-butène comprenant la formation de 2,3-diméthyl-1-butène à partir de propène en présence d'un catalyseur de dimérisation et l'isomérisation du 2,3-diméthyl-1-butène formé en présence d'au moins un acide choisi parmi l'acide sulfurique ayant une concentration d'au moins 70% et des acides sulfoniques.

3. Procédé selon la revendication 1 ou 2, dans lequel ledit acide sulfonique est au moins un acide sulfonique choisi parmi des acides sulfoniques aliphatiques, des acides sulfoniques aromatiques et des acides sulfoniques halogénés.

4. Procédé selon la revendication 3, dans lequel l'acide sulfonique aromatique est l'acide benzènesulfonique ou l'acide p-toluènesulfonique.

5. Procédé selon la revendication 1 ou 2, dans lequel ledit acide est utilisé dans une quantité de 0,001 à 5% en poids rapportés au 2,3-diméthyl-1-butène.

6. Procédé selon la revendication 1 ou 2, dans lequel au moins un solvant est choisi parmi des hydrocarbures aromatiques, des hydrocarbures aliphatiques et des hydrocarbures halogénés.

7. Procédé selon la revendication 6, dans lequel la quantité dudit solvant est comprise entre 0,01 et 50 parties en poids pour une partie de 2,3-diméthyl-1-butène.

8. Procédé selon la revendication 1 ou 2, dans lequel la température de la réaction d'isomérisation est comprise entre -30 et 100°C.

9. Procédé selon la revendication 1 ou 2, dans lequel le procédé est réalisé en présence d'acide sulfurique ayant une concentration d'au moins 70%.

10. Procédé selon la revendication 2, dans lequel ledit catalyseur de dimérisation est désactivé après la dimérisation de propène pour former du 2,3-diméthyl-1-butène et le 2,3-diméthyl-1-butène est ensuite isomérisé.

11. Procédé selon la revendication 2 ou 10, dans lequel ledit catalyseur de dimérisation est un catalyseur homogène.

12. Procédé selon la revendication 11, dans lequel ledit catalyseur homogène est au moins un catalyseur qui comprend un composé de nickel choisi parmi (1) un catalyseur à base de complexe de π-allylnickel/halogénure organique d'aluminum/composé de phosphore trivalent, (2) un catalyseur à base de sel de nickel/halogénure organique d'aluminium /composé de phosphore trivalent, (3) un catalyseur à base de composé de nickel/trialkylaluminium/composé de phosphore trivalent/phénol halogéné/eau, (4) un catalyseur à base de composé de nickel/trialkylaluminium/composé de phosphore trivalent/alcool isopropylique fluoré, (5) un catalyseur à base de composé de nickel/trialkylaluminium/composé de phosphore trivalent/phénol halogéné/acide sulfonique ou sulfate de dialkyle, (6) un catalyseur à base de composé de nickel/trialkylaluminium/composé de phosphore trivalent/alcool isopropylique fluoré/acide sulfonique ou sulfate de dialkyle, et (7) un catalyseur à base de composé de nickel/ trialkylaluminium/composé de phosphore trivalent/phénol halogéné/eau/acide sulfonique.

**13.** Procédé selon la revendication 10, dans lequel un désactiveur dudit catalyseur est au moins un composé choisi parmi des alcools, des phénols, l'ammoniaque et des amines.